# EUROPEAN PATENT SPECIFICATION

(11) **EP 3 930 706 B1**
(45) Date of publication and mention of the grant of the patent: **22.05.2024**
(21) Application number: 20714424.7
(22) Date of filing: 28.02.2020
(51) Int. Cl.: A61K 31/351, A61P 3/10

(54) **USE OF SOTAGLIFLOZIN FOR THE TREATMENT OF PATIENTS WITH TYPE 1 DIABETES MELLITUS**
VERWENDUNG VON SOTAGLIFLOZIN ZUR BEHANDLUNG VON PATIENTEN MIT DIABETES MELLITUS TYP 1
UTILISATION DE SOTAGLIFLOZINE POUR LE TRAITEMENT DE PATIENTS ATTEINTS DE DIABÈTE SUCRÉ DE TYPE 1

(30) Priority: 01.03.2019 US 201962812338 P
(43) Date of publication of application: 05.01.2022
(73) Proprietor: Lexicon Pharmaceuticals, Inc., The Woodlands, TX 77381 (US)
(72) Inventor: BANKS, Phillip, Dublin, Ohio 43017 (US); SAWHNEY, Sangeeta, Chapel Hill, North Carolina 27514 (US)
(74) Representative: Abel & Imray LLP
(86) International application number: PCT/US2020/020295
(87) International publication number: WO 2020/180647

(56) References cited:
- Pablo Lapuerta ET AL: "Development of sotagliflozin, a dual sodium-dependent glucose transporter 1/2 inhibitor", Diabetes & Vascular Disease Research, 1 March 2015 (2015-03-01), pages 101-110, XP55434899, London, England DOI: 10.1177/1479164114563304 Retrieved from the Internet: URL:http://journals.sagepub.com/doi/pdf/10 .1177/1479164114563304
- MOTTALIB ADHAM ET AL: "Weight Management in Patients with Type 1 Diabetes and Obesity", CURRENT DIABETES REPORTS, CURRENT SCIENCE, PHILADELPHIA, VA, US, vol. 17, no. 10, 23 August 2017 (2017-08-23), pages 1-9, XP036328512, ISSN: 1534-4827, DOI: 10.1007/S11892-017-0918-8 [retrieved on 2017-08-23]
- ARTHUR T. SANDS ET AL: "Sotagliflozin, a Dual SGLT1 and SGLT2 Inhibitor, as Adjunct Therapy to Insulin in Type 1 Diabetes", DIABETES CARE, vol. 38, no. 7, 6 June 2015 (2015-06-06), pages 1181-1188, XP55695661, US ISSN: 0149-5992, DOI: 10.2337/dc14-2806

## Description

### 1. FIELD OF THE INVENTION

The invention relates to the use of sotagliflozin to improve glycemic control in patients with type 1 diabetes mellitus with a Body Mass Index (BMI) ≥ 27 kg/m².

### 2. BACKGROUND OF THE INVENTION

Sotagliflozin is the generic name for (2S,3R,4R,5S,6R)-2-(4-chloro-3-(4-ethoxybenzyl)phenyl)-6-(methylthio)tetrahydro-2H-pyran-3,4,5-triol: 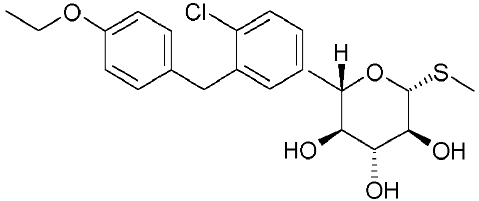 which is described, for example in WO 2008/109591. Sotagliflozin is a dual inhibitor of the sodium glucose co-transporters 1 and 2 (SGLT1 and SGLT2). SGLT1 is the primary transporter for absorption of glucose and galactose in the intestine. SGLT2 is a transporter that reabsorbs glucose from the renal filtrate and prevents the loss of glucose in the urine. Due to its mechanism of action, sotagliflozin can be useful for the treatment of diabetes, in particular type 1 diabetes mellitus.

Type 1 diabetes (T1D) is a chronic condition characterised by an autoimmune destruction of β-cells in the pancreas, ultimately resulting in an absolute insulin deficiency and the patient's life-long dependence on exogenous insulin substitution. Chronically elevated blood glucose levels usually accompany insulin self-management and can cause damage to blood vessels leading to long-term micro- and macrovascular complications, which account for most of the increased morbidity and mortality associated with T1D. Incidence of T1D varies widely globally, ranging from 0.1/100,000 per year in China and Venezuela to 36.5/100,000 per year in Finland (Karvonen M, Viik-Kajander M, Moltchanova E, Libman, I, LaPorte R, et al. Incidence of childhood type 1 diabetes worldwide. Diabetes Mondiale (DiaMond) Project Group. doi:10.2337/diacare .23.10.1516 Diabetes Care October 2000 vol. 23 no. 10 1516-1526). Every year in the United States, on average more than 15,000 young people under the age of 20 years are diagnosed with T1D (Centers for Disease Control, 2011, National diabetes fact sheet: national estimates and general information on diabetes and prediabetes in the United States, 2011. Atlanta, GA: U.S. Department of Health and Human Services, Centers for Disease Control and Prevention, 2011). Maintaining glycemic control without hypoglycaemia is a key therapeutic goal in T1D, to foster both near-term and long-term health. To date, this has only been achieved by maintaining strict dietary discipline with vigilant glucose monitoring linked to appropriate exogenous insulin use throughout the day. Chronic insulin use to maintain tight glycemic control presents significant challenges for patients, both logistically and medically, with constant approximations and adjustments made to accommodate various meals and activity levels. The challenges and inherent imperfections of such calculations contribute to the tendency of many T1D patients to maintain blood glucose levels at the high end or above recommended glycemic targets, in order to avoid the immediate dangers of hypoglycaemia, ultimately at the expense of suffering long-term consequences of chronic high blood sugar.

Despite the broad availability of insulin therapy for T1D, there remain large segments of the patient population for whom adequate glycemic control is not consistently achieved. Indeed, the majority of adult patients with T1D are under-served by currently available therapies, and most are not able to consistently achieve HbA1C goals on insulin therapy alone, even with optimal disease management. For example, according to the T1D registry (T1D Registry. https://t1dexchange.org/pages/clinic-registry/. Accessed March 16, 2015.), (-30% <15 years old, - 70% <30 years old), of the 26,127 total registrants, 23% have poor glycemic control with inability to achieve a hemoglobin A1C (A1C) <9.0%, while -17% of registrants with the same standards of care (SOC) and medical providers are able to attain an A1C <7.0%. It is likely that a spectrum of pathophysiological and socioeconomic drivers contribute to the wide disparity in glycemic control that is observed, and that certain groups within the T1D population find it particularly difficult to maintain glycemic control with currently available therapies.

Given the challenges associated with improving treatment outcomes for groups that have a history of especially poor control, there is a need to identify a safe and simple pharmacological treatment that can consistently improve glycemic control when added to insulin regimens. Importantly, attention must also be given to the goal of improving the quality of life in T1D patients by reducing insulin injection burden and the need for strict glucose monitoring. Therefore, there is still an unmet need of adjunct therapy for patients with T1D, in particular those who have an inadequate glycemic control despite insulin therapy.

During phase II trials, sotagliflozin has demonstrated an ability to significantly improve glycemic control in patients with type 1 diabetes, in particular by achieving a reduction in A1C (Lapuerta et al., Diabetes & Vascular Disease Research 2015, Vol. 12(2) 101-110).

Though patients with T1DM have traditionally been thought to have normal body mass index (BMI), current research has shown that the trend of increasing obesity prevalence has increased at a faster rate in patients with T1DM compared to the general population. Currently, up to 50% of patients with T1DM are either overweight or obese (Ryden A, Sorstadius E, Bergenheim K et al. The Humanistic Burden of Type 1 Diabetes Mellitus in Europe: Examining Health Outcomes and the Role of Complications. PLoS One. 2016;11:e0164977; Mottalib A, Kasetty M, Mar JY, Elseaidy T, Ashrafzadeh S, Hamdy O. Weight management in patients with type 1 diabetes and Obesity. Curr Diab Rep. 2017;17:92.). Various reasons can explain this increasing incidence, but data show that intensified insulin therapy aimed at tightening glycemic control is frequently associated with weight gain. Therefore, the unmet need in patients with T1DM is even greater in those who are overweight and obese, in whom ideal treatment should improve haemoglobin A1c (A1C) while inducing weight loss.

### 3. SUMMARY OF THE INVENTION

This invention is directed to the use of sotagliflozin or a pharmaceutically acceptable salt thereof for use in improving glycemic control in patientshaving type 1 diabetes mellitusand a Body Mass Index (BMI) ≥ 27 kg/m², wherein the sotagliflozin is orally administered once daily in an amount of 200mg.

### 4. DETAILED DESCRIPTION OF THE INVENTION

This invention is based on the discovery that sotagliflozin improves glycemic control in adults with type 1 diabetes mellitus with a Body Mass Index (BMI) ≥ 27 kg/m². Sotagliflozin, which is chemically named (2S,3R,4R,5S,6R)-2-(4-chloro-3-(4-ethoxybenzyl)phenyl)-6-(methylthio)tetrahydro-2H-pyran-3,4,5-triol, has the structure shown below:

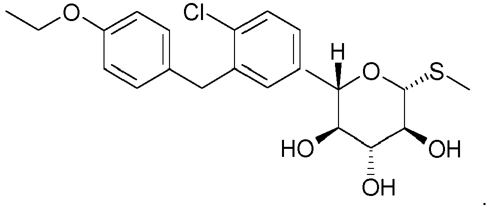

Thus, one embodiment of the invention is sotagliflozin or a pharmaceutically acceptable salt thereof for use in improving glycemic control in patientshaving type 1 diabetes mellitusand a Body Mass Index (BMI) ≥ 27 kg/m², in particular in patients who have failed to achieve adequate glycemic control despite optimal insulin therapy.

Particular embodiments of the invention relate to sotagliflozin or a pharmaceutically acceptable salt thereof for use to improve glycemic control in adultshaving type 1 diabetes mellitusand a Body Mass Index (BMI) ≥ 27 kg/m² as an adjunct to insulin therapy, in particular in patients who have failed to achieve adequate glycemic control despite optimal insulin therapy.

In some embodiments, sotagliflozin is administered before the first meal of the day. To the extent that the following examples relate to subject matter outside the scope of the claims, they are provided for comparative purposes only.

### 4.1. Example 1: inTandem 1 and inTandem 2 studies

The efficacy, relative to a placebo, of sotagliflozin as adjunct therapy in adult patients with Type 1 Diabetes Mellitus who have inadequate glycemic control with insulin therapy, was evaluated in multicenter, double-blind clinical studies inTandem 1 and inTandem 2 with random distribution in three groups of treatment (group treated with sotagliflozin 200 mg daily dose, group treated with sotagliflozin 400 mg daily dose, and group treated with a placebo) of patients.

InTandem 1 was conducted in North America and inTandem 2 was conducted in Europe and Israel. The selected patients were adult patients diagnosed with T1D who have inadequate glycemic control with insulin therapy (administered by multiple daily injections [MDI] or continuous subcutaneous insulin infusion [CSII]). Patients continued treatment with insulin(s) or insulin analog(s) during the studies. The patient recruitment was carried out by taking into account the following inclusion criteria: Adult patients 18 years and older with a diagnosis of T1D made at least 1 year prior to informed consent; Patients are being treated with insulin(s) or insulin analog(s) delivered via CSII or MDI where the method of insulin delivery has not changed from CSII to MDI or vice-versa in the 3 months prior to the Screening Visit; At the Screening Visit, A1C must be 7.0% to 11.0%, inclusive; Must be willing and able to perform self-monitored blood glucose (SMBG) and complete the study diary as required per protocol; Females of childbearing potential must use an adequate method of contraception to avoid pregnancy throughout the duration of the study and for 30 days after the last dose of study drug. Females of childbearing potential include any female who has experienced menarche and who has not undergone successful surgical sterilization (hysterectomy, bilateral tubal ligation, or bilateral oophorectomy) or is not postmenopausal. Postmenopause is defined as no menses for ≥12 months without another cause. For females with questionable menopausal history (eg, irregular menstrual periods and age >40 years) a documented serum follicle-stimulating hormone (FSH) level must be ≥30 mIU/mL; Females of childbearing potential must have a negative serum or urine pregnancy test prior to the start of study drug. In the case of positive urine pregnancy testing, a negative serum sample for pregnancy testing, to confirm that the patient is not pregnant, must be obtained prior to start of study.

Patients were excluded from the study using the criteria that included: 1) Therapies and/or medications: Use of antidiabetic agent other than insulin(s) or insulin analog(s) at the time of screening (any medication other than insulin or insulin analog used for treatment of T1D must be washed out for at least 8 weeks prior to the Screening Visit); Any prior exposure to sotagliflozin; Use of sodium-glucose cotransporter (SGLT) inhibitors within 8 weeks prior to Screening; Chronic systemic corticosteroid use, defined as any dose of systemic corticosteroid taken for more than 4 consecutive weeks within the 6 months prior to the Screening Visit. 2) Diabetes-related conditions: Type 2 diabetes mellitus (T2DM), or severely uncontrolled T1D as determined by the Investigator; History of severe hypoglycemic event within 1 month prior to the Screening Visit; History of DKA within 1 month prior to Screening Visit, or more than 2 episodes within 6 months prior to the Screening Visit; History of nonketotic hyperosmolar state within 6 months prior to the Screening Visit. 3) Laboratory Results: Estimated glomerular filtration rate (eGFR) <45 mL/min/1.73 m2 at Screening, as determined by the 4 variable Modification of Diet in Renal Disease (MDRD) equation; Fasting triglycerides (TG) >600 mg/dL (>6.77 mmol/L). Note: Patients who fail Screening based on this criterion must have their fasting status verified, may have TG-lowering medications adjusted, and be reevaluated during the Screening Period; Abnormal liver function at Screening defined as any of the following: aspartate aminotransferase (AST) >2X upper limit of the normal reference range (ULN), alanine aminotransferase (ALT) >2X ULN, serum total bilirubin (TB) >1.5X ULN; Screening beta-hydroxy butyrate (BHB) >0.6 mmol/L. 4) Reproductive status: Females who are pregnant or breastfeeding or intend to be during the course of the study. 5) Gastrointestinal (Gl)/hepatic: By known history, serologic evidence of current infectious liver disease (hepatitis A, B, or C), including anti hepatitis A virus (immunoglobulin M), hepatitis B surface antigen, or anti hepatitis C virus; Difficulty swallowing such that the patient cannot take the study drug; History of pancreatitis within 12 months of screening, or any prior history of recurrent pancreatitis. 6) Renal: Initiation of chronic dialysis within 30 days prior to the Screening Visit or expected to occur within 180 days after the Screening Visit; Renal disease that required treatment with immunosuppressive therapy, or a history of dialysis or renal transplant; History of hereditary glucose-galactose malabsorption or primary renal glucosuria. 7) Cardiovascular: New York Heart Association Class III or IV heart failure within 3 months prior to Screening Visit; Hypertensive urgency or emergency within 30 days prior to randomization; Patients with unstable/symptomatic or life-threatening arrhythmia or heart block; Patient has had any of the following within 3 months prior to the Screening Visit: Hospitalization due to unstable angina, Myocardial infarction (MI), Coronary artery bypass graft (CABG) or percutaneous transluminal coronary angioplasty, or Transient ischemic attack (TIA) or significant cerebrovascular disease. 8) Hematologic: History of hemoglobinopathies (sickle cell anemia, thalassemia major, sideroblastic anemia) or other disorder that may interfere with A1C determination; Donation or loss of >400 mL of blood or blood product(s) within 8 weeks prior to Screening. 9) Immune system: Known severe immunocompromised status, including, but not limited to, patients who have undergone organ transplantation. 10) Malignancy or active treatment for malignancy within 5 years prior to the Screening Visit. 11) Current eating disorder or increase or decrease of weight within the 12 weeks prior to Screening by more than 10%. 12) Known allergies, hypersensitivity, or intolerance to sotagliflozin or any inactive component of sotagliflozin or placebo unless the reaction is deemed irrelevant to the study by the Investigator. 13) Administration of any other investigational drug or participation in an interventional clinical research study within 30 days or 5 half-lives (whichever is longer) of planned Screening Visit. 14) History of alcohol or illicit drug abuse (using Diagnostic and Statistical Manual of Mental Disorders, 5th edition criteria) within 12 months prior to the Screening Visit. 15) Patient is a study coordinator, employee of an Investigator or Investigator's site, or immediate family member of any of the aforementioned. 16) Any condition that, in the opinion of the Investigator, may render the patient unable to complete the study. 17) The presence of a clinically significant medical history, physical examination, or laboratory finding that, in the opinion of the Investigator or the Sponsor, may interfere with any aspect of study conduct or interpretation of results.

The studies were conducted as follows:
- Screening Period (up to 2 weeks)
- Initiation of insulin optimization (starting at Week -6);
- Single-blind placebo Run-in Period (starting at Week -2): Patients will take single-blind placebo tablets orally during the single-blind placebo Run-in Period;
- 24-Week Double-blind Core Treatment Period: Following completion of the single-blind placebo Run-in Period, eligible patients will enter the 24-week, double-blind Core Treatment Period. Patients will be randomly assigned 1:1:1 in parallel among the 3 treatment groups: sotagliflozin 200 mg, 400 mg, or placebo;
- 28-Week Double-blind Long-term Extension Period: The purpose of the 28-week double-blind LTE Period is to provide data on the risk/benefit of sotagliflozin treatment over time, and to assess the durability of efficacy in patients treated with sotagliflozin over time.

Sotagliflozin and placebo were administered orally using tablets:
- Sotagliflozin 400 mg as two (2) 200-mg tablets, once daily, before the first meal of the day
- Sotagliflozin 200 mg as one (1) 200-mg tablet plus 1 placebo tablet, once daily, before the first meal of the day
- Placebo as two (2) placebo tablets (identical to sotagliflozin tablets in appearance), once daily, before the first meal of the day

The primary endpoint of these studies was the change from Baseline to Week 24 in glycated hemoglobin A1C of either dose of sotagliflozin (400 mg or 200 mg) treatment group compared to placebo. Other endpoints included the change from baseline of sotagliflozin versus placebo on the following:
- Proportion of patients with A1C <7.0% and no episode of
- severe hypoglycemia and no episode of diabetic ketoacidosis (DKA)
- Body weight
- Bolus insulin dose
- Fasting plasma glucose (FPG)
- Diabetes Treatment Satisfaction Questionnaire status
- (DTSQs) score,
- and 2-item Diabetes Distress Screening Scale (DDS2) questionnaire score

Efficacy analyses were based on the mITT (modified Intent-to-Treat) population. Primary efficacy analyses were conducted based on data collected during the Core Treatment Period (Baseline through and including the Week 24 Visit assessments). Table 1 shows that sotagliflozin achieved its primary endpoint by leading to statistically significant lower A1C levels for both doses when compared to placebo in the pooled inTandem1/inTandem2 study results after the core treatment period and the long-term extension period. Table 2 presents data from an analysis in subgroups categorized by baseline BMI <27 kg/m² and ≥27 kg/m² in the pooled inTandem1/inTandem2 study (LS=Lean square mean, SE= standard error).

**Table 1. Pooled Efficacy and Safety Results from Randomization to Week 52 on a Background of Optimized Insulin Therapy inTandem1 and 2)**

| | | **PBO** | **SOTA 200 mg** | **SOTA 400 mg** |
|---|---|---|---|---|
| | | n = 526 | n = 524 | n = 525 |
| **Baseline (BL) Characteristics** | | | | |
| **Age** (mean [s.d.]) | | 42.5 (13.3) | 44.4 (13.7) | 44.0 (13.4) |
| **HbA1c** (%) (mean [s.d.]) | | 7.66 (0.81) | 7.68 (0.77) | 7.64 (0.78) |
| **Weight** (kg) (mean [s.d.]) | | 84.25 (17.56) | 84.46 (18.13) | 84.23 (18.11) |
| **Body mass index** (kg/m²) (mean [s.d.]) | | 28.54 (5.28) | 28.89 (5.56) | 28.74 (5.18) |

| **Efficacy through 52 Weeks** | | | | |
|---|---|---|---|---|
| **HbA1c change from Baseline** | | | | |
| | **Week 24 Treatment Comparison** | | | |
| | LS Mean (SE), %, p | N/A | -0.36 (p<0.001) | -0.38 (p<0.001) |
| | **Week 52 Treatment Comparison** | | | |
| | LS Mean (SE), %, p | N/A | -0.23 (p<0.001) | -0.32 (p<0.001) |
| **Weight (kg)** | | | | |
| | **-Percent Change** (%) **from Baseline at Week 24** | | | |
| | LS Mean (SE), p | 0.47 (0.137), p<0.001 | -1.70 (0.137), p<0.001 | -2.55 (0.137), p<0.001 |
| | Treatment Comparison vs. PBO LS Mean (SE), p | N/A | -2.17 (0.188), p<0.001 | -3.02 (0.188), p<0.001 |
| | **Percent Change** (%) **from Baseline at Week 52** | | | |
| | LS Mean (SE), p | 0.91 (0.209), p<0.001 | -2.31 (0.208), p<0.001 | -3.33 (0.209), p<0.001 |
| | Treatment Comparison vs. PBO LS Mean (SE), p | N/A | -3.21 (0.291), p<0.001 | -4.24 (0.291), p<0.001 |
| **No Weight Gain at Week 52** (SOTA vs. PBO) | | | | |
| | **Percentage HbA1c** <7.0% | 9.1 | 21.8 | 26.1 |
| | Absolute difference in %, *p*^{a} | N/A | 12.6 (p<0.001) | 17.0 (p<0.001) |
| | **Percentage with absolute reduction in HbA1c ≥0.5%** | | | |
| | Absolute difference in %, *p* ^{a} | 8.8 | 23.7 | 28.8 |
| | | N/A | 14.9 (p<0.001) | 20.0 (p<0.001) |

| **Patients with Safety Event through 52 Weeks** | | | | |
|---|---|---|---|---|
| Any TEAE, n (%) | | 374 (71.1) | 393 (75.0) | 390 (74.3) |
| DKA, ^{b,c} n (%) | | 1 (0.2) | 15 (2.9) | 20 (3.8) |
| Severe hypoglycemia, ^{b} n (%) | | 39 (7.4) | 30 (5.7) | 23 (4.4) |
| Diarrhea, ^{d} n (%) | | 27 (5.1) | 34 (6.5) | 46 (8.8) |
| Genital mycotic infection, n (%) | | 15 (2.9) | 48 (9.2) | 63 (12.0) |
| BMI, body mass index; DKA, diabetic ketoacidosis; LS, least square mean; N/A, Not applicable; SE, standard error; SH, severe hypoglycemia; TEAE, Treatment-emergent adverse event. ^{a} *p*-value based on Wald test. ^{b} Number of patients with ≥1 positively adjudicated event. ^{c} DKA cases were adjudicated as "yes with certainty," "yes, probably," "no, unlikely," "no with certainty," "unclassifiable," and "insufficient data." Positively adjudicated cases were classified as either "with certainty" or "probably." ^{d} Discontinuation of drug due to diarrhea was: 0.4% PBO, 0.4% SOTA 200 mg, and 0.8% SOTA 400 mg. | | | | |

**Table 2. Benefits by baseline BMI < or ≥27 kg/m² - Pooled lnTandem1/lnTandem2 (miTT population) - Week 24**

| | | **Patients with Baseline BMI <27 kg/m²** | | | **Patients with Baseline BMI ≥27 kg/m²** | | |
|---|---|---|---|---|---|---|---|
| | | **Placebo (N=228)** | **200 mg (N=219)** | **400 mg (N=212)** | **Placebo (N=298)** | **200 mg (N=305)** | **400 mg (N=313)** |
| **A1C (%)** | | | | | | | |
| | Mean baseline | 7.72 | 7.62 | 7.65 | 7.62 | 7.72 | 7.63 |
| | LS Mean change (SE) | -0.06 (0.047) | -0.38 (0.047) | -0.34 (0.048) | -0.04 (0.034) | -0.43 (0.034) | -0.50 (0.034) |
| | LS Mean Difference (SE) | - | -0.32 (0.062) | -0.27 (0.063) | - | -0.39 (0.046) | -0.45 (0.045) |
| | 95% CLs for Difference | | [-0.44, -0.20] | [-0.40, -0.15] | - | [-0.48, -0.30] | [-0.54, -0.36] |
| | p-value | | <0.001 | <0.001 | - | <0.001 | <0.001 |

| **Body weight (kg)** | | | | | | | |
|---|---|---|---|---|---|---|---|
| | Mean baseline | 71.24 | 70.21 | 70.31 | 94.20 | 94.68 | 93.66 |
| | LS Mean change (SE) | 0.63 (0.174) | -1.43 (0.177) | -1.91 (0.181) | 0.34 (0.198) | -1.93 (0.196) | -2.98 (0.193) |
| | LS Mean Difference (SE) | - | -2.06 (0.240) | -2.55 (0.243) | - | -2.27 (0.272) | -3.32 (0.270) |
| | 95% CLs for Difference | - | [-2.53, -1.59] | [-3.02, -2.07] | - | [-2.81, -1.74] | [-3.85, -2.79] |
| | p-value | - | <0.001 | <0.001 | - | <0.001 | <0.001 |

| **SBP (mmHg)** | | | | | | | |
|---|---|---|---|---|---|---|---|
| | Mean baseline | 119.0 | 117.2 | 117.9 | 124.3 | 124.6 | 123.6 |
| | LS Mean change (SE) | 0.1 (0.72) | -3.0 (0.74) | -3.4 (0.75) | -1.6 (0.65) | -2.9 (0.64) | -4.0 (0.64) |
| | LS Mean Difference (SE) | - | -3.1 (0.97) | -3.5 (0.98) | - | -1.3 (0.87) | -2.5 (0.87) |

| | | **Patients with Baseline BMI <27 kg/m²** | | | **Patients with Baseline BMI ≥27 kg/m²** | | |
|---|---|---|---|---|---|---|---|
| | | **Placebo (N=228)** | **200 mg (N=219)** | **400 mg (N=212)** | **Placebo (N=298)** | **200 mg (N=305)** | **400 mg (N=313)** |
| | 95% CLs for Difference | - | [-5.0, -1.2] | [-5.4, -1.6] | - | [-3.0, 0.4] | [-4.2, -0.8] |
| | p-value | - | 0.002 | <0.001 | - | 0.13 | 0.005 |

| **Time in range (%)** | | **N=35** | **N=30** | **N=31** | **N=58** | **N=59** | **N=65** |
|---|---|---|---|---|---|---|---|
| | Mean baseline | 55.298 | 52.273 | 51.393 | 50.683 | 52.155 | 50.317 |
| | LS Mean change (SE) | -2 -2.170 (2.9600) | -0.532 (2.9540) | 4.164 (2.9683) | -1.917 (2.2503) | 6.254 (2.1983) | 13.133 (2.0244) |
| | LS Mean Difference (SE) | - | 1.638 (3.8450) | 6.335 (3.8492) | - | 8.171 (2.9713) | 15.051 (2.8562) |
| | 95% CLs for Difference | - | [-6.070, 9.346] | [-1.384, 14.054] | - | [2.296, 14.046] | [9.403, 20.699] |
| | p-value | - | 0.67 | 0.11 | - | 0.007 | <0.001 |

| **Fasting plasma glucose (mg/dL)** | | | | | | | |
|---|---|---|---|---|---|---|---|
| | Mean baseline | 156.6 | 153.6 | 157.7 | 157.3 | 163.5 | 156.3 |
| | LS Mean change (SE) | 4.8 (4.14) | -10.8 (4.22) | -11.5 (4.32) | 6.4 (3.36) | -9.3 (3.33) | -18.6 (3.28) |
| | LS Mean Difference (SE) | - | -15.7 (5.69) | -16.4 (5.78) | - | -15.7 (4.57) | -25.0 (4.53) |
| | 95% CLs for Difference | - | [-26.9, -4.5] | [-27.8, -5.0] | - | [-24.7, -6.7] | [-33.9, -16.1] |
| | p-value | - | 0.006 | 0.005 | - | <0.001 | <0.001 |

| | | **Patients with Baseline BMI <27 kg/m²** | | | **Patients with Baseline BMI ≥27 kg/m²** | | |
|---|---|---|---|---|---|---|---|
| | | **Placebo (N=228)** | **200 mg (N=219)** | **400 mg (N=212)** | **Placebo (N=298)** | **200 mg (N=305)** | **400 mg (N=313)** |
| **2-hour postprandial plasma glucose (mg/dL)** | | **N=35** | **N=30** | **N=31** | **N=58** | **N=59** | **N=65** |
| | Mean baseline | 238.9 | 207.6 | 211.3 | 224.7 | 213.5 | 208.9 |
| | LS Mean change (SE) | 16.5 (18.86) | -49.5 (17.63) | -69.6 (17.34) | -17.7 (11.05) | -36.8 (11.72) | -39.5 (10.72) |
| | LS Mean Difference (SE) | - | -66.0 (20.22) | -86.1 (21.27) | - | -19.0 (14.71) | -21.7 (13.93) |
| | 95% CLs for Difference | - | [-106.4,-25.6] | [-128.6, -43.6] | - | [-48.1, 10.1] | [-49.3, 5.8] |
| | p-value | - | 0.002 | <0.001 | - | 0.20 | 0.12 |

| **DTSQ (score)** | | | | | | | |
|---|---|---|---|---|---|---|---|
| | Mean baseline | 28.2 | 28.2 | 28.9 | 28.7 | 28.4 | 28.8 |
| | LS Mean change (SE) | 0.0 (0.32) | 1.7 (0.33) | 1.5 (0.33) | -0.3 (0.27) | 2.3 (0.26) | 2.2 (0.26) |
| | LS Mean Difference (SE) | - | 1.7 (0.42) | 1.5 (0.43) | - | 2.6 (0.35) | 2.6 (0.35) |
| | 95% CLs for Difference | - | [0.9, 2.6] | [0.7, 2.3] | - | [1.9, 3.3] | [1.9, 3.3] |
| | p-value | - | <0.001 | <0.001 | - | <0.001 | <0.001 |

| **DDS2 (score)** | | | | | | | |
|---|---|---|---|---|---|---|---|
| | Mean baseline | 5.2 | 5.3 | 5.3 | 5.1 | 5.4 | 5.1 |
| | LS Mean change (SE) | 0.1 (0.12) | -0.2 (0.13) | -0.4 (0.13) | 0.1 (0.10) | -0.5 (0.10) | -0.5 (0.10) |
| | LS Mean Difference (SE) | - | -0.4 (0.16) | -0.5 (0.16) | - | -0.6 (0.13) | -0.7 (0.13) |
| | 95% CLs for Difference | - | [-0.7, -0.0] | [-0.8, -0.2] | - | [-0.9, -0.3] | [-0.9, -0.4] |
| | p-value | - | 0.028 | 0.002 | - | <0.001 | <0.001 |

At baseline, no difference was observed among the treatment groups in A1C while, in addition to a higher BMI, overweight and obese patients with BMI ≥27 kg/m² tended to have higher baseline systolic blood pressure (SBP). Overall, at Week 24, the efficacy of sotagliflozin versus placebo was more pronounced in the overweight and obese subgroups with BMI ≥27 kg/m², especially for the measurements of A1C, body weight, time in range, Diabetes Treatment Satisfaction Questionnaire (DTSQ) and 2-item Diabetes Distress Screening Scale (DDS2). Using the same statistical models, tests of a treatment by subgroup interaction were associated with p-values <0.05 for the A1C, body weight, and DTSQ change from baseline scores. This means that for these variables, the sotagliflozin efficacy was systematically better in patients with BMI ≥27 kg/m2 compared to the lower BMI subgroup. The difference tended to be greater in the 400 mg group for A1C, body weight and time in range. The decrease in fasting plasma glucose was greater in the overweight and obese patients for the 400 mg dose only. The decrease in SBP and 2-hour postprandial glucose tended to be lower in these patients.

A similar trend in findings was observed at Week 52, data from which is presented in Table 3. Table 4 presents data from a risk analysis, from which it is clear that the compound is both safe and effective.

**Table 3. Benefits by baseline BMI < or ≥27 kg/m² - Pooled lnTandem1/lnTandem2 (miTT population) - Week 52**

| | | **Patients with Baseline BMI <27 kg/m²** | | | **Patients with Baseline BMI ≥27 kg/m²** | | |
|---|---|---|---|---|---|---|---|
| | | **Placebo (N=228)** | **200 mg (N=219)** | **400 mg (N=212)** | **Placebo (N=298)** | **200 mg (N=305)** | **400 mg (N=313)** |
| **A1C (%)** | | | | | | | |
| | Mean baseline | 7.72 | 7.62 | 7.65 | 7.62 | 7.72 | 7.63 |
| | LS Mean change (SE) | 0.01 (0.057) | -0.19 (0.057) | -0.20 (0.057) | -0.00 (0.042) | -0.24 (0.041) | -0.38 (0.040) |
| | LS Mean Difference (SE) | - | -0.20 (0.078) | -0.21 (0.079) | - | -0.24 (0.056) | -0.38 (0.055) |
| | 95% CLs for Difference | | [-0.35, -0.05] | [-0.37, -0.06] | - | [-0.35, -0.13] | [-0.49, -0.27] |
| | p-value | | 0.010 | 0.007 | - | <0.001 | <0.001 |

| **Body weight (kg)** | | | | | | | |
|---|---|---|---|---|---|---|---|
| | Mean baseline | 71.24 | 70.21 | 70.31 | 94.20 | 94.68 | 93.66 |
| | LS Mean change (SE) | 0.69 (0.228) | -1.57 (0.230) | -1.72 (0.238) | 0.85 (0.258) | -2.16 (0.254) | -3.61 (0.249) |
| | LS Mean Difference (SE) | - | -2.26 (0.317) | -2.41 (0.324) | - | -3.01 (0.357) | -4.46 (0.353) |
| | 95% CLs for Difference | - | [-2.88, -1.64] | [-3.04, -1.77] | - | [-3.71, -2.31] | [-5.15, -3.76] |
| | p-value | - | <0.001 | <0.001 | - | <0.001 | <0.001 |

| **SBP (mmHg)** | | | | | | | |
|---|---|---|---|---|---|---|---|
| | Mean baseline | 119.0 | 117.2 | 117.9 | 124.3 | 124.6 | 123.6 |
| | LS Mean change (SE) | 2.7 (0.78) | -1.4 (0.79) | -1.0 (0.81) | 0.4 (0.67) | -1.7 (0.66) | -3.2 (0.65) |
| | LS Mean Difference (SE) | - | -4.1 (1.05) | -3.7 (1.08) | - | -2.1 (0.90) | -3.6 (0.89) |
| | 95% CLs for Difference | - | [-6.2, -2.0] | [-5.8, -1.6] | - | [-3.9, -0.4] | [-5.3, -1.9] |
| | p-value | - | <0.001 | <0.001 | - | 0.018 | <0.001 |

**Table 4. Main risks by baseline BMI < or ≥27 kg/m² - Pooled InTandem1/inTandem2 - Week 52**

| | | **Patients with Baseline BMI <27 kg/m²** | | | **Patients with Baseline BMI ≥27 kg/m²** | | |
|---|---|---|---|---|---|---|---|
| | | **Placebo (N=228)** | **200 mg (N=219)** | **400 mg (N=212)** | **Placebo (N=298)** | **200 mg (N=305)** | **400 mg (N=313)** |
| **Positively Adjudicated Severe Hypoglycemia** | | | | | | | |
| | Events [n (%) n] | 17 (7.5) 21 | 17 (7.8) 43 | 11 (5.2) 16 | 22 (7.4) 29 | 13 (4.3) 25 | 12 (3.8) 17 |
| | Events per Subject per Year | 0.104 | 0.214 | 0.087 | 0.107 | 0.089 | 0.058 |
| | EAIR per 1,000 Subject-years (95% CL) | 83.86 (44.00,123.73) | 84.77 (44.48,125.07) | 59.50 (24.34,94.67) | 81.03 (47.17,114.90) | 46.51 (21.23,71.80) | 41.06 (17.83,64.29) |
| | Risk Difference of EAIR (95% CL) Minus Placebo | - | 0.91 (-55.78,57.60) | -24.36 (-77.52,28.80) | - | -34.52 (-76.78,7.74) | -39.98 (-81.04,1.09) |
| | Relative Risk of EAIR (95% CL) Versus Placebo | - | 1.01 (0.51,2.00) | 0.71 (0.32,1.51) | - | 0.57 (0.28,1.14) | 0.51 (0.24,1.02) |

| **Documented hypoglycemia ≤55 mg/dL** | | | | | | | |
|---|---|---|---|---|---|---|---|
| | Events per Subject per Year | 20.44 | 16.86 | 15.74 | 17.87 | 13.41 | 14.45 |
| | Event rate (95% CL) | 20.27 (17.54, 23.01) | 16.68 (14.37, 18.99) | 16.99 (14.56,19.41) | 17.99 (15.86, 20.13) | 13.75 (12.12, 15.37) | 14.57 (12.88,16.26) |
| | Event rate difference (95% CL) | - | -3.59 (-7.17, -0.01) | -3.29 (-6.94, 0.37) | - | -4.25 (-6.93, -1.57)) | -3.42 (-6.14, -0.70) |
| | p value | - | 0.0491 | 0.0781 | - | 0.0019 | 0.0138 |

| **Positively Adjudicated Diabetic Ketoacidosis** | | | | | | | |
|---|---|---|---|---|---|---|---|
| | Events [n (%) n] | 0 | 7 (3.2) 7 | 9 (4.2) 9 | 1 (0.3) 1 | 8 (2.6) 9 | 11 (3.5) 11 |
| | Events per Subject per Year | 0 | 0.035 | 0.049 | 0.004 | 0.032 | 0.038 |
| | EAIR per 1,000 Subject-years (95% CL) | 0 | 34.91 (9.05,60.77) | 48.68 (16.88,80.49) | 3.68 (0.00,10.90) | 28.62 (8.79,48.46) | 37.64 (15.39,59.88) |
| | Risk Difference of EAIR (95% CL) Minus Placebo | - | 34.91 (9.05,60.77) | 48.68 (16.88,80.49) | - | 24.94 (3.83,46.05) | 33.95 (10.57,57.34) |
| | Relative Risk of EAIR (95% CL) Versus Placebo | - | NC | NC | - | 7.77 (1.24,173.82) | 10.22 (1.74,221.94) |

| **Genital Mycotic Infections (male)** | | **N=116** | **N=108** | **N=99** | **N=155** | **N=157** | **N=154** |
|---|---|---|---|---|---|---|---|
| | Events [n (%) n] | 2 (1.7) 2 | 2 (1.9) 2 | 9 (9.1) 10 | 1 (0.6) 2 | 6 (3.8) 7 | 7 (4.5) 10 |
| | Events per Subject per Year | 0.019 | 0.020 | 0.113 | 0.014 | 0.048 | 0.068 |
| | EAIR per 1,000 Subject-years (95% CL) | 19.19 (0.00,45.79) | 20.37 (0.00,48.60) | 101.91 (35.33,168.49) | 6.89 (0.00,20.39) | 41.05 (8.20,73.89) | 47.74 (12.37,83.10) |

| | | **Patients with Baseline BMI <27 kg/m²** | | | **Patients with Baseline BMI ≥27 kg/m²** | | |
|---|---|---|---|---|---|---|---|
| | | **Placebo (N=228)** | **200 mg (N=219)** | **400 mg (N=212)** | **Placebo (N=298)** | **200 mg (N=305)** | **400 mg (N=313)** |
| | Risk Difference of EAIR (95% CL) Minus Placebo | - | 1.18 (-37.61,39.96) | 82.71 (11.02,154.41) | - | 34.16 (-1.35,69.67) | 40.85 (2.99,78.70) |
| | Relative Risk of EAIR (95% CL) Versus Placebo | - | 1.06 (0.11,10.19) | 5.31 (1.26,36.06) | - | 5.96 (0.88,138.01) | 6.93 (1.07,157.36) |

| **Genital Mycotic Infections (female)** | | **N=112** | **N=111** | **N=113** | **N=143** | **N=148** | **N=159** |
|---|---|---|---|---|---|---|---|
| | Events [n (%) n] | 3 (2.7) 9 | 8 (7.2) 10 | 19 (16.8) 31 | 9 (6.3) 14 | 32 (21.6) 41 | 28 (17.6) 48 |
| | Events per Subject per Year | 0.091 | 0.098 | 0.321 | 0.111 | 0.308 | 0.330 |
| | EAIR per 1,000 Subject-years (95% CL) | 30.45 (0.00,64.92) | 78.17 (24.00,132.34) | 196.79 (108.30,285.28) | 71.25 (24.70,117.80) | 240.02 (156.86,323.19) | 192.26 (121.05,263.48) |
| | Risk Difference of EAIR (95% CL) Minus Placebo | - | 47.71 (-16.49,111.91) | 166.34 (71.38,261.30) | - | 168.78 (73.47,264.08) | 121.01 (35.94,206.09) |
| | Relative Risk of EAIR (95% CL) Versus Placebo | - | 2.57 (0.70,11.96) | 6.46 (2.09,27.41) | - | 3.37 (1.65,7.46) | 2.70 (1.30,6.04) |

| **Diarrhea** | | | | | | | |
|---|---|---|---|---|---|---|---|
| | Events [n (%) n] | 7 (3.1) 7 | 18 (8.2) 23 | 19 (9.0) 28 | 20 (6.7) 25 | 16 (5.2) 22 | 27 (8.6) 37 |
| | Events per Subject per Year | 0.035 | 0.115 | 0.151 | 0.092 | 0.079 | 0.127 |
| | EAIR per 1,000 Subject-years (95% CL) | 34.53 (8.95,60.11) | 89.76 (48.29,131.23) | 102.78 (56.56,148.99) | 73.67 (41.38,105.95) | 57.25 (29.20,85.30) | 92.38 (57.53,127.23) |
| | Risk Difference of EAIR (95% CL) Minus Placebo | - | 55.23 (6.51,103.95) | 68.25 (15.42,121.07) | - | -16.42 (-59.19,26.35) | 18.71 (-28.79,66.22) |
| | Relative Risk of EAIR (95% CL) Versus Placebo | - | 2.60 (1.11,6.68) | 2.98 (1.28,7.60) | - | 0.78 (0.40,1.51) | 1.25 (0.70,2.27) |

The numerically lower risk of severe hypoglycemia was observed for both sotagliflozin doses in overweight and obese patients with baseline BMI ≥27 kg/m², but only for 400 mg in patients with baseline BMI<27 kg/m². In addition, the risk difference versus placebo was greater in patients with baseline BMI ≥27 kg/m². The event rates of documented hypoglycemia ≤55 mg/dL were numerically lower for both sotagliflozin doses in both BMI subgroups. The difference in event rate versus placebo was greater and nominally significant in patients with a baseline BMI ≥27 kg/m² in the sotagliflozin 200 mg and 400 mg treatment groups.

Although the risk of DKA for sotagliflozin versus placebo was increased in both BMI subgroups, various measurements of comparative risk (e.g., exposure-adjusted incidence rate and risk difference versus placebo) tended to be lower in patients with baseline BMI ≥27 kg/m² in both sotagliflozin 200 mg and 400 mg groups.

For both male and female patients, the risk difference versus placebo in genital mycotic infections does not show any consistent trend in patients with a baseline BMI ≥27 kg/m² compared to leaner patients. No increased risk of diarrhea compared to placebo was observed in patients with a baseline BMI ≥27 kg/m² at any doses, and the risk was lower in these patients compared to leaner patients.

### 4.2. Example 2: inTandem 3 Study

An additional phase 3, randomized, double-blind, placebo-controlled, parallel-group, multicenter study, inTandem 3, was conducted, in order to evaluate the net clinical benefit of sotagliflozin as adjunct to insulin therapy in Type 1 Diabetes.

The selected patients were adult patients diagnosed with T1D who have inadequate glycemic control with insulin therapy (administered by multiple daily injections [MDI] or continuous subcutaneous insulin infusion [CSII]). Patients continued treatment with insulin(s) or insulin analog(s) during the studies. The patient recruitment was carried out by taking into account the following inclusion criteria: Adult patients 18 years and older with a diagnosis of T1D made at least 1 year prior to informed consent; Patients are being treated with insulin(s) or insulin analog(s); Non-fast acting insulin dose is stable (±20%) for 2 weeks prior to the Screening Visit; At the Screening Visit, A1C must be between 7.0% and 11.0%, inclusive 6) BMI ≥18.5 kg/m2; Must be willing and able to perform SMBG and complete the study diary as required per protocol; Females of childbearing potential must use an adequate method of contraception to avoid pregnancy throughout the duration of the study and for 30 days after the last dose of study drug. Females of childbearing potential include any female who has experienced menarche and who has not undergone successful surgical sterilization (hysterectomy, bilateral tubal ligation, or bilateral oophorectomy) or is not postmenopausal. Postmenopause is defined as no menses for ≥12 months without another cause. For females with questionable menopausal history (e.g., irregular menstrual periods and age >40 years) a documented serum follicle-stimulating hormone (FSH) level must be ≥30 mIU/mL; Females of childbearing potential must have a negative serum or urine pregnancy test prior to the start of study drug. In the case of positive urine pregnancy testing, a negative serum sample for pregnancy testing, to confirm that the patient is not pregnant, must be obtained prior to start of study.

Patient were excluded from the study using criteria that included the following: 1) Therapies and/or medications: Use of antidiabetic agent other than insulin(s) or insulin analog(s) at the time of screening (any medication other than insulin or insulin analog used for treatment of T1D must be washed out for at least 8 weeks prior to the Screening Visit); Any prior exposure to sotagliflozin; Use of sodium-glucose cotransporter (SGLT) inhibitors within 8 weeks prior to Screening. Note: Patients taking an SGLT inhibitor may have that prohibited medication stopped and may be considered for entry into the study if they have not been taking the prohibited medication for at least 8 weeks prior to Screening; Chronic systemic corticosteroid use, defined as any dose of systemic corticosteroid taken for more than 4 consecutive weeks within the 6 months prior to the Screening Visit. Note: Topical, inhaled, ocular, or nasal sprays containing corticosteroids are allowed. 2) Diabetes-related conditions: Type 2 diabetes mellitus or severely uncontrolled T1D as determined by the Investigator; History of severe hypoglycemic event within 1 month prior to the Screening Visit; History of DKA or nonketotic hyperosmolar state within 1 month of Screening OR ≥2 episodes of DKA or nonketotic hyperosmolar state within 6 months of Screening. 3) Laboratory results: Estimated glomerular filtration rate (eGFR) <45 mL/min/1.73 m2 at Screening, as determined by the 4 variable Modification of Diet in Renal Disease (MDRD) equation; Fasting triglycerides >600 mg/dL (>6.77 mmol/L). Note: Patients who fail Screening based on this criterion must have their fasting status verified, may have triglyceride-lowering medications adjusted, and be reevaluated during Screening; Abnormal liver function at Screening defined as any of the following: aspartate aminotransferase (AST) >2X upper limit of the normal reference range (ULN), alanine aminotransferase (ALT) >2X ULN, serum total bilirubin (TB) >1.5X ULN; Screening beta-hydroxy butyrate (BHB) >0.6 mmol/L. 4) Reproductive status: Females who are pregnant or breastfeeding or intend to be during the course of the study. 5) Gastrointestinal/hepatic: By known history, serologic evidence of current infectious liver disease (hepatitis A, B, or C), including anti hepatitis A virus (immunoglobulin M), hepatitis B surface antigen, or anti hepatitis C virus. Note: Patients with isolated positive hepatitis B surface antibody may be included; Difficulty swallowing such that the patient cannot take the study drug; History of pancreatitis within 12 months of screening, or any prior history of recurrent pancreatitis. 6) Renal: Initiation of chronic dialysis within 30 days prior to the Screening Visit or expected to occur within 180 days after the Screening Visit; Renal disease that required treatment with immunosuppressive therapy, or a history of dialysis or renal transplant; History of hereditary glucose-galactose malabsorption or primary renal glucosuria. 7) Cardiovascular: New York Heart Association Class III or IV heart failure within 3 months prior to Screening Visit; Hypertensive urgency or emergency within 30 days prior to randomization; Patients with unstable/symptomatic or life-threatening arrhythmia or heart block; Patient has had any of the following within 3 months prior to the Screening Visit: Hospitalization due to unstable angina; Myocardial infarction (MI); Coronary artery bypass graft (CABG) or percutaneous transluminal coronary angioplasty; Transient ischemic attack (TIA) or significant cerebrovascular disease. 8) Hematologic: History of hemoglobinopathies (sickle cell anemia, thalassemia major, sideroblastic anemia) or other disorder that may interfere with A1C determination; Donation or loss of >400 mL of blood or blood product(s) within 8 weeks prior to Screening. 9) Immune system: Known severe immunocompromised status, including, but not limited to, patients who have undergone organ transplantation; 10) Malignancy or active treatment for malignancy (i.e., radiation or chemotherapy, including monoclonal antibodies) within 5 years prior to the Screening Visit; 11) Current eating disorder or increase or decrease of weight within the 12 weeks prior to Screening by more than 10%; 12) Known allergies, hypersensitivity, or intolerance to sotagliflozin or any inactive component of sotagliflozin or placebo unless the reaction is deemed irrelevant to the study by the Investigator; 13) Administration of any other investigational drug or participation in an interventional clinical research study within 30 days or 5 half-lives (whichever is longer) of planned Screening Visit; 14) History of alcohol or illicit drug abuse (using Diagnostic and Statistical Manual of Mental Disorders, 5th edition criteria) within 12 months prior to the Screening Visit. 15) Patient is a study coordinator, employee of an Investigator or Investigator's site, or immediate family member of any of the aforementioned. 16) Any condition that, in the opinion of the Investigator, may render the patient unable to complete the study. 17) The presence of a clinically significant medical history, physical examination, or laboratory finding that, in the opinion of the Investigator or the Sponsor, may interfere with any aspect of study conduct or interpretation of results. The Investigator or the Sponsor will supply justification for exclusion, if applicable.

The duration of participation was of approximately 32 weeks, as follows: Screening Period (up to 2 weeks); 2-week single-blind placebo Run-in Period; 24-week double-blind Treatment Period. The primary objective of this study was to demonstrate the superiority of sotagliflozin 400 mg versus placebo in the proportion of patients with glycosylated hemoglobin A1C (A1C) <7.0% at Week 24 and no episode of severe hypoglycemia and no episode of diabetic ketoacidosis (DKA) after randomization. Secondary objectives of this study included to evaluate the change from Baseline of sotagliflozin versus placebo on the following: A1C; Body weight; Systolic blood pressure (SBP); and Bolus insulin dose.

As shown in Table 5, sotagliflozin reached its primary endpoint. Table 6 provides data form an analysis in subgroups categorized by baseline BMI <27 kg/m² and ≥27 kg/m² in the inTandem3 study, which shows the same trend as inTandem1/inTandem2 study.

**Table 5. Patients Achieving Glycated Hemoglobin <7.0% Without Severe Hypoglycemia or Diabetic Ketoacidosis (DKA) and Those With Glycated Hemoglobin ≥7.0% With Severe Hypoglycemia (SH) or Diabetic Ketoacidosis**

| **Characteristic** | | | **Sotagliflozin (n=699)** | **Placebo (n=703)** | **LS mean difference from placebo (95% CI) P value** | |
|---|---|---|---|---|---|---|
| Primary endpoint: Patients achieving glycated hemoglobin <7% without DKA and no severe hypoglycemia | | | | | | |
| | All patients-no. (%) | | 200 (28.6) | 107 (15.2) | 13.4% (8.97 to 17.81) | |
| | | | | | <0.001 | |
| | Insulin pump users (n=555)^{†}-no. (%) | | 88 (32.0) | 45 (16.1) | 15.9% (8.58 to 23.38) | |
| | | | | | <0.001 | |
| | Nonpump users (n=847)^{†}-no. (%) | | 112 (26.4) | 62 (14.7) | 11.8% (6.14 to 17.38) | |
| | | | | | <0.001 | |

| Secondary endpoints | | | | | | |
|---|---|---|---|---|---|---|
| | Glycated hemoglobin-% | | | | | |
| | | Baseline mean ± SD | 8.26 ± 0.965 | 8.21 ± 0.921 | | |
| | | Week 24 no. patients | 627 | 628 | | |
| | | Week 24 mean ± SD | 7.41 ± 0.95 | 7.88 ± 1.03 | | |
| | | Week 24 LS mean change from baseline ± SE (95% CI) | -0.79 ± 0.03 (-0.85, -0.73) | -0.33 ± 0.03 (-0.39 to -0.27) | -0.46 ±0.04 (-0.54 to -0.38) | |
| | | P value | <0.001 | <0.001 | <0.001 | |

| | Weight-kg | | | | | |
|---|---|---|---|---|---|---|
| | | Baseline mean ± SD | 82.40 ± 17.13 | 81.55 ± 17.03 | | |
| | | Week 24 no. patients | 630 | 633 | | |
| | | Week 24 mean ± SD | 80.19 ± 17.02 | 82.70 ± 17.51 | | |
| | | Week 24 LS mean change from baseline ± SE (95% CI) | -2.21 ± 0.12 (-2.45 to -1.97) | 0.77 ± 0.012 (0.53 to 1.01) | -2.98 ± 0.166 (-3.31 to -2.66) | |
| | | P value | <0.001 | <0.001 | <0.001 | |

| **Characteristic** | | | **Sotagliflozin (n=699)** | **Placebo (n=703)** | **LS mean difference from placebo (95% CI) P value** | |
|---|---|---|---|---|---|---|
| | Systolic blood pressure in patients with SBP ≥130 mm Hg at baseline (n=406)-mm Hg^{‡} | | | | | |
| | | Baseline mean ± SD | 140.5 ± 8.70 | 139.9 ±9.53 | | |
| | | Week 16 no. patients | 186 | 192 | | |
| | | Week 16 mean ± SD | 131.8 ± 12.02 | 134.3 ± 12.77 | | |
| | | Week 16 LS mean change from baseline ± SE (95% CI) | -9.2 ± 0.92 (-11.0 to -7.4) | -5.7 ± 0.90 (-7.5 to -3.9) | -3.5 ± 1.11 (-5.7 to -1.3) | |
| | | P value | <0.001 | <0.001 | 0.002 | |

| | Daily bolus insulin dose-IU/day | | | | | |
|---|---|---|---|---|---|---|
| | | Baseline mean ± SD | 27.34 ± 16.97 | 28.72 ± 19.04 | | |
| | | Week 24 no. patients | 619 | 626 | | |
| | | Week 24 mean ± SD | 23.90 ± 15.76 | 28.17 ± 18.55 | | |
| | | Week 24 LS mean change from baseline ± SE (95% CI) | -3.93 ± 0.47 (-4.85 to -3.01) | -1.09 ± 0.47 (-2.00 to -0.17) | -2.84 ± 0.61 (-4.05 to -1.64) | |
| | | P value | <0.001 | 0.020 | <0.001 | |
| | | Week 24 LS mean percent change from baseline ± SE | -5.71 ± 2.29 | 6.62 ± 2.72 | -12.32 ± 2.98 | |
| | | (95% CI) | (-10.19 to -1.22) | (2.16, 11.08) | (-18.17 to -6.48) | |
| | | P value | 0.013 | 0.004 | <0.001 | |

**Table 6. Benefits by baseline BMI < or ≥27 kg/m² - InTandem3 (mITT population) - Week 24**

| | | **Patients with Baseline BMI <27 kg/m²** | | **Patients with Baseline BMI ≥27 kg/m²** | |
|---|---|---|---|---|---|
| | | **Placebo (N=333)** | **400 mg (N=320)** | **Placebo (N=370)** | **400 mg (N=379)** |
| **A1C** (%) | | | | | |
| | Mean baseline | 8.21 | 8.34 | 8.21 | 8.19 |
| | LS Mean change (SE) | -0.35 (0.050) | -0.72 (0.051) | -0.32 (0.066) | -0.86 (0.065) |
| | LS Mean Difference (SE) | - | -0.37 (0.068) | - | -0.54 (0.051) |
| | 95% CLs for Difference | - | [-0.51, -0.24] | - | [-0.64, -0.44] |
| | p-value | - | <0.001 | - | <0.001 |

| **Body weight (kg)** | | | | | |
|---|---|---|---|---|---|
| | Mean baseline | 69.69 | 69.65 | 92.22 | 93.17 |
| | LS Mean change (SE) | 0.75 (0.155) | -1.71 (0.157) | 0.61 (0.280) | -2.80 (0.277) |
| | LS Mean Difference (SE) | - | -2.47 (0.213) | - | -3.41 (0.247) |
| | 95% CLs for Difference | - | [-2.88, -2.05] | - | [-3.90, -2.93] |
| | p-value | - | <0.001 | - | <0.001 |

| **SBP (mmHg)** | | | | | |
|---|---|---|---|---|---|
| | Mean baseline | 118.8 | 118.1 | 124.5 | 125.2 |
| | LS Mean change (SE) | 1.8(0.69) | -1.7 (0.69) | -0.6 (1.22) | -3.8(1.19) |
| | LS Mean Difference (SE) | - | -3.4 (0.92) | - | -3.2 (0.79) |
| | 95% CLs for Difference | - | [-5.3, -1.6] | - | [-4.7, -1.6] |
| | p-value | - | <0.001 | - | <0.001 |

| **Fasting plasma glucose (mg/dL)** | | | | | |
|---|---|---|---|---|---|
| | Mean baseline | 163.9 | 167.6 | 163.0 | 163.0 |
| | LS Mean change (SE) | 11.3 (4.26) | -10.4 (4.30) | 0.8 (6.46) | -23.5 (6.34) |
| | LS Mean Difference (SE) | - | -21.7 (5.78) | - | -24.3 (4.69) |
| | 95% CLs for Difference | - | [-33.0, -10.3] | - | [-33.5, -15.1] |
| | p-value | - | <0.001 | - | <0.001 |

In the InTandem 3 study, no treatment group difference was observed in baseline A1C between overweight and obese patients with BMI ≥27 kg/m² compared to those with BMI <27 kg/m², but patients with the higher BMI category also tended to have higher baseline SBP. Overall, the efficacy of sotagliflozin compared to placebo in the overweight and obese subgroup tended to have had a more favorable effect (greater mean reduction from baseline) on A1C and body weight.

In addition to a higher BMI, overweight and obese patients are more likely to have other co-morbidities (e.g., older age, longer disease duration), and therefore present a higher medical need that may not be addressed by insulin alone and may benefit from sotagliflozin. These data provide surprising clinical evidence for a more favorable benefit-risk profile in patients with baseline BMI ≥27kg/m² based on greater A1C reduction, greater weight loss, and favorable trends for less SH (severe hypoglycemia) and less DKA than subjects with baseline BMI <27kg/m².

## Claims

1. Sotagliflozin or a pharmaceutically acceptable salt thereof for use in improving glycemic control in a patient having type 1 diabetes and a Body Mass Index (BMI) ≥ 27 kg/m², wherein the sotagliflozin is orally administered once daily in an amount of 200 mg.

2. Sotagliflozin or a pharmaceutically acceptable salt thereof for use according to claim 1, wherein the patient is taking insulin.

3. Sotagliflozin or a pharmaceutically acceptable salt thereof for use according to claim 1, wherein the patient has failed to achieve adequate glycemic control despite optimal insulin therapy.

4. Sotagliflozin or a pharmaceutically acceptable salt thereof for use according to any of claims 1-3, wherein sotagliflozin is administered as an adjunct to insulin therapy.

## Patentansprüche

1. Sotagliflozin oder ein pharmazeutisch annehmbares Salz davon zur Verwendung zur Verbesserung der glykämischen Kontrolle bei einem Patienten mit Typ-1-Diabetes und einem Body-Mass-Index (BMI) ≥ 27 kg/m², wobei das Sotagliflozin einmal täglich in einer Menge von 200 mg oral verabreicht wird.

2. Sotagliflozin oder ein pharmazeutisch annehmbares Salz davon zur Verwendung gemäß Anspruch 1, wobei der Patient Insulin einnimmt.

3. Sotagliflozin oder ein pharmazeutisch annehmbares Salz davon zur Verwendung gemäß Anspruch 1, wobei der Patient trotz optimaler Insulintherapie keine ausreichende glykämische Kontrolle erreicht hat.

4. Sotagliflozin oder ein pharmazeutisch annehmbares Salz davon zur Verwendung gemäß einem der Ansprüche 1-3, wobei Sotagliflozin als Ergänzung zur Insulintherapie verabreicht wird.

## Revendications

1. Sotagliflozine ou sel pharmaceutiquement acceptable de celle-ci à utiliser pour améliorer la régulation glycémique chez un patient ayant un diabète de type 1 et un indice de masse corporelle (IMG) ≥ 27 kg/m², la sotagliflozine étant administrée par voie orale une fois par jour en une quantité de 200 mg.

2. Sotagliflozine ou sel pharmaceutiquement acceptable de celle-ci à utiliser selon la revendication 1, dans lequel le patient prend de l'insuline.

3. Sotagliflozine ou sel pharmaceutiquement acceptable de celle-ci à utiliser selon la revendication 1, dans lequel le patient n'a pas réussi à obtenir une régulation glycémique adéquate en dépit d'une insulinothérapie optimale.

4. Sotagliflozine ou sel pharmaceutiquement acceptable de celle-ci à utiliser selon l'une quelconque des revendications 1 à 3, la sotagliflozine étant administrée comme traitement complémentaire à l'insulinothérapie.
